# EUROPEAN PATENT SPECIFICATION

(11) **EP 2 029 030 B1**
(45) Date of publication and mention of the grant of the patent: **10.11.2010**
(21) Application number: 07732604.9
(22) Date of filing: 30.04.2007
(51) Int. Cl.: A61B 17/02, A61B 19/00

(54) **SURGICAL APPARATUS**
OPERATIONSGERÄT
APPAREIL CHIRURGICAL

(30) Priority: 29.04.2006 GB 0608561
(43) Date of publication of application: 04.03.2009
(73) Proprietor: Comis Orthopaedics Limited, Magna Way Rotherham S60 1FB (GB)
(72) Inventor: CHANA, Gursharan Singh, Sutton Coldfield B74 2SL (GB); SLOSS, Christopher John, Magna Way, Rotherham S60 1FB (GB)
(74) Representative: Lock, Howard John
(86) International application number: PCT/GB2007/001570
(87) International publication number: WO 2007/125348

(56) References cited:
- WO-A-00/66008
- WO-A-2004/047650
- WO-A-2005/037089
- WO-A-2005/092206
- US-A1- 2004 193 018
- US-A1- 2004 199 055
- US-B1- 6 190 312
- US-B1- 6 431 025

## Description

This invention relates to surgical apparatus. More particularly, but not exclusively the invention relates to surgical apparatus for carrying out minimally invasive surgery. Embodiments of the invention relate to surgical apparatus for carrying out hip surgery, such as minimally invasive hip surgery.

Retraction systems for carrying out hip surgery, particularly minimally invasive hip surgery, require the use of significant force on the retractors to expose the acetabulum and femur, to allow for their preparation, and for the implantation of a prosthesis. In addition, some systems require assistance to hold the retractors in position.

Specification No. WO 2004/047650A2 discloses surgical instruments for accessing and stabilizing a localized portion of a beating heart and maintaining open a surgical space according to the preamble of claim 1.

According to one aspect of this invention there is provided a surgical apparatus comprising a support member having first and second opposite sides; first and second retractors on the support member; a first mounting arrangement on the first side, and a second mounting arrangement on the second side; the first mounting arrangement being configured to mount the first retractor on the first side, wherein at least the first retractor is movable towards and away from the second retractor, and the second mounting arrangement being configured to mount the second retractor on the second side; and each mounting arrangement comprising a securing assembly to secure the first and second retractors to the first and second mounting arrangements respectively; wherein each securing assembly comprises a fixed member; a movable member to which the first or second retractor can be secured; a fastener extending through the fixed member, the fastener cooperatively engaging the fixed member; characterised by urging means on the fastener to urge the movable member into engagement with the fixed member.

The first retractor or mounting arrangement may be linearly movable towards and away from the second retractor or mounting arrangement. The first retractor or mounting arrangement may be pivotally movable relative to the support member. The second retractor or mounting arrangement may be pivotally movable relative to the support member.

The first and second retractors or mounting arrangements may be arranged in opposed positions relative to one another on the support member. Desirably, the first and second retractors or mounting arrangements face each other.

The support member may define an access region through which surgery can be carried out. The support member may surround the aforesaid access region. The retractors may extend through the access region. The support member may comprise first and second opposite sides upon which the first and second retractors or mounting arrangements are respectively mounted.

Each of the first and second sides may comprise a central region and opposite end regions extending transverse to the central region. The opposite end regions of each of the first and second sides may be angled relative to the central region at an angle in the range of 20° to substantially 30° to the central region. The opposite end regions may be angled substantially 30° to the central region. This provides the advantage in one embodiment that the support member conforms generally to the region of the body upon which the surgery is carried out.

The support member may comprise opposite third and fourth sides. The third and fourth sides may extend between the first and second sides to connect the first side to the second side, thereby providing a generally rectangular profile of the support member. In one embodiment, the support member may have a generally square configuration.

The third and fourth sides may each include a movable securing member to secure a respective surgical member thereto. The third and fourth sides may include serrations to hold the securing member in a selected position on the respective third or fourth side. Each securing member may include a securing formation to which a connecting element can be attached to connect the respective surgical member to the securing member. The securing formation may comprise an upstanding projection. The connecting element may be elongate and may be flexible. In one embodiment, the connecting element may comprise a chain. Each surgical member may comprise a Hohman retractor.

The first and second retractors may be provided with a respective manipulating member tn move the first and second retractors tn the desired orientations

Each surgical member may comprise a respective manipulating member to move the respective surgical member to its desired position. Each manipulating member may comprise a handle.

The first and second mounting arrangements may be fixedly mounted un the support member, for example by welding, riveting or by casting.

The fastener may comprise a shaft, having a threaded region, and a head. The head may be provided at one end of the shaft. The threaded region of the shaft may be provided at the opposite end region of the shaft. The fastener may comprise a bolt.

The fastener may co-operatively engage the movable member. The movable member may be threaded to threadably engage the fastener.

The movable member may have interlocking formations. The fixed member may have corresponding interlocking formations to co-operatively engage the interlocking formations on the movable member. Thus, when the movable member is engaged on the fastener, the interlocking formations on the movable member may engage the interlocking formations on the fixed member.

In one embodiment, when the fastener tightens the movable member against the fixed member, the interlocking formations prevent relative rotation of the movable member and the fixed member.

The urging means may comprise a spring, such as a coil spring.

The movable member may include a securing formation to secure the respective first or second retractor onto the movable member. The first and second retractors may each comprise a securing element to engage the respective movable member. The securing element may be slidably mounted on the securing formation. The securing formation and the securing element may be correspondingly configured to substantially prevent relative rotation of the movable member and the respective retractor.

In one embodiment, the securing formation may comprise a generally rectangular outer profile of the movable member. The securing element may have a generally rectangular inner profile, said inner profile of the securing element corresponding to the outer profile of the securing formation.

Each of the movable members may be pivotally movable relative to the respective first or second fixed members to arrange the respective first or second retractors at a desired angle relative to the support member.

The threaded engagement of the fastener with the respective movable member may lock the movable member against the fixed member, thereby preventing pivoting of the respective first or second retractor.

The first mounting arrangement may comprise a displacement mechanism to move the first retractor towards and away from the second retractor. The displacement mechanism may comprise a displacement member to which the respective fixed member is attached.

The displacement member may be elongate and may comprise a rack. The displacement mechanism may comprise a housing, through which the displacement member movably extends.

The first mounting arrangement may include a driver to drive the displacement member. The driver may comprise a rotatable head and a cog in engagement with the displacement member. The displacement member may comprise a plurality of teeth arranged to co-operate with the cog. The displacement member and the cog may comprise a rack and pinion arrangement.

A locking member may be provided to lock the displacement member to the housing. In a first embodiment, the locking member may comprise a threaded member threadably receivable in the housing to engage the mounting member. The locking member may further include a rotatable head mounted on the threaded member to rotate the threaded member.

In a second embodiment, the locking member may comprise a rocker assembly, which may comprise a depressible element for engaging the displacement members. The depressible element may be movable between a locking position and a release position by pressing thereon.

The depressible element may pivot about a pivot member between the locking and the release positions.

The depressible element may be capable of being pressed at a first region on one side of the pivot member to move the depressible element to the locking position. The depressible element may be capable of being pressed at a second region on the opposite side of the pivot member to move the depressible element to the release position.

The rocker assembly may comprise an urging arrangement to urge the depressible element to the locking position. The urging arrangement may comprise a spring, such as a leaf spring. The urging arrangement may be provided on the depressible element, and may engage the mounting arrangement.

The mounting arrangement may include a reaction portion which the urging arrangement can engage. The urging arrangement may produce a reaction against the reaction portion when the depressible element is moved to the release position. The urging arrangement may react against the reaction portion to urge the depressible element to the locking position.

An exemplary method of using a surgical apparatus as described above comprises arranging the first and second retractors on the member, inserting the retractors in an incision in a body and moving the first retractor towards and/or away from the second retractor to retract a portion of the body upon which surgery is being effected.

The method may comprise linearly moving the first retractor towards and/or away from the first retractor.

The method may comprise pivoting the first retractor relative to the support member. The method may comprise pivoting the second retractor relative to the support member.

The method may comprise arranging the first and second retractors in opposed positions relative to each other. The method may comprise arranging the first and second retractors such that they face each other.

The support member may define an access region, and the method may comprise carrying out the surgery through the access region.

The method may comprise mounting the retractors on first and second opposite sides of the support member.

In one embodiment, the method may comprise securing the surgical members to a respective one of third and fourth opposite sides of the support member. Each surgical member may comprise a Hohman retractor.

A retractor may be provided comprising a securing element for securing the retractor to a support member and a blade extending from the securing element.

The securing element may comprise a generally rectangular profile for co-operation with a correspondingly shaped securing part on the support member. The rectangular profile may be an inner rectangular profile.

The blade may have a longitudinal axis and may be curved about the longitudinal axis.

Embodiments of the invention will now be described by way of example only, with reference to the accompanying drawings, in which:
Fig 1 is a perspective view of a first embodiment of a surgical apparatus;
Fig 1A is a side view of a support member forming part of the surgical apparatus shown in Fig 1;
Fig 2 is a sectional view of a mounting arrangement forming part of the surgical apparatus shown in Fig 1;
Figs 3 and 4 are perspective views of two retractors;
Figs 5 and 6 are perspective views of first and second Hohman retractors;
Fig 7 is a perspective view of a second embodiment of a surgical apparatus;
Fig 8 is a sectional view through the mounting arrangement shown in Fig. 7 showing the rocker assembly; and
Fig 9 is a perspective view of a third Hohman retractor

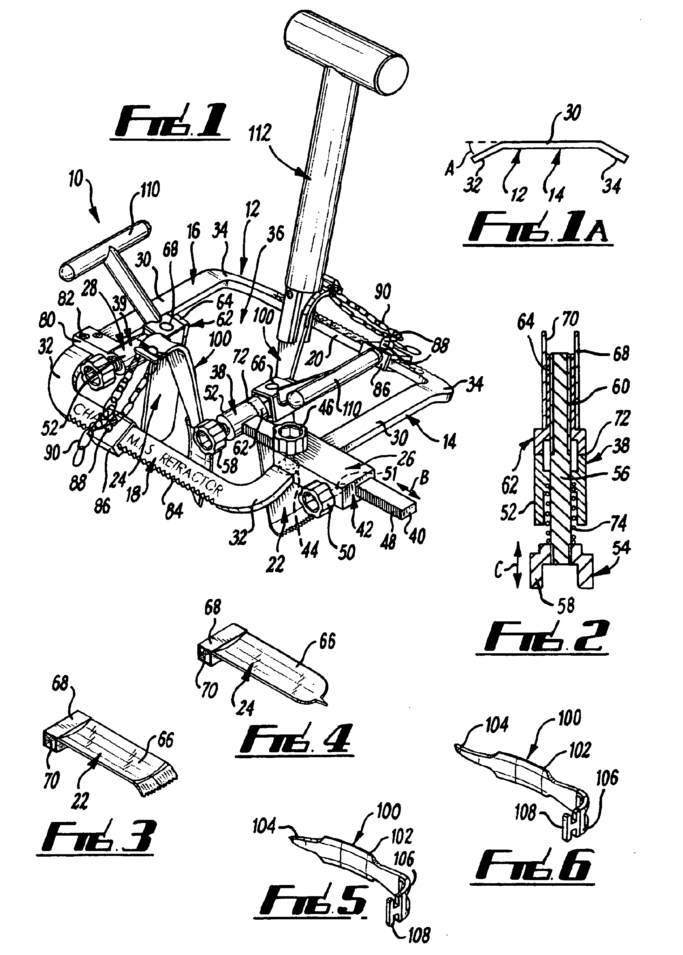

Referring to Fig 1, there is shown a first embodiment of a surgical apparatus 10 comprising a support member in the form of a generally square frame 12 comprising first and second opposite sides 14, 16 and third and fourth opposite sides 18, 20. The third and fourth opposite sides 18, 20 connect the first and second opposite sides 14, 16 to each other.

A first retractor 22 is mounted on the first side 14, and a second retractor 24 is mounted on the second side 16.

A first mounting arrangement 26 mounts the first retractor 22 on the first side 14, and a second mounting arrangement 28 mounts the second retractor 24 on the second side 16.

Referring to Fig 1A there is shown a side view of the support member 12 in which the first side member 14 faces out of the paper. The second side member 16 and the third and fourth side members 18, 20 are hidden behind the first side member 14. The first side member 12 comprises a central region 30 and angled end regions 32, 34 which extend from the central region 30 at an angle A of substantially 30°. It will be appreciated that the second side member 16 also includes a central region 30 and first and second end regions 32, 34 extending at an angle of substantially 30° to the central region 30.

The feature of the end regions 32, 34 extending at an angle to the central region 30 of each of the first and second side members 14, 16 provides the advantage in this embodiment that the support member 12 follows the contours of the body on which the operation is effected.

The support member 12 defines a generally square central space 36 via which the operation can be carried out.

The first mounting arrangement 26 comprises a displacement member in the form of an elongate rack 40. The elongate rack 40 extends through a housing 42 fixedly mounted on the first side 14. A pinion 44, shown in broken lines is provided in the housing 42, and connected to a wheel 46 by means of which the pinion 44 can be rotated.

The rack 40 is provided with a plurality of teeth 48 arranged adjacent one another along its length, and the pinion 44 includes a plurality of corresponding teeth arranged adjacent one another around the circumference of the pinion 44.

The teeth on the pinion 44 engage the teeth 48 on the rack 40 and upon rotation of the wheel 46 the rack 40 can be moved linearly in and out of a housing 42 in the directions shown by double headed arrow B in Fig 1. Thus, the first retractor 22 can be moved towards and away from the second retractor 24 by rotating the wheel 46. A locking member 50 having a locking shaft 51, shown in broken lines in Fig 1, is provided on the side of the housing 42. The locking member 50 is in the form of a bolt or screw and is threadably mounted on the housing 42. When the locking member 50 is screwed into the housing 42, it engages the rack 40 to lock the rack 40 in its position.

The first mounting arrangement 26 also includes a first securing assembly 38 to which the respective first or second retractor 22, 24 is secured. The first securing assembly 38 is shown in section in Fig 2 and comprises a fixed member 52 through which a fastener in the form of a bolt 54 extends. The bolt 54 comprises a shaft 56, extending through the fixed member 52, and a head 58 to enable the bolt 54 to be rotated. The shaft 56 is provided with threads 60 along at least a part of its length along the end region opposite the head 58.

A movable member 62 threadably engages the shaft 56 such that when the bolt 54 is screwed into the fixed member 52, the fixed member 52 is tightened against the movable member 62. The movable member 62 comprises a generally rectangular outer profile 64 which corresponds to the inner profile of a securing element 66 of the retractor 22, as explained below.

Referring to Figs 3 and 4, there is shown examples of the first and second retractors 22, 24. It will be appreciated that either of the retractors 22, 24 could be used on either of the mounting arrangements 26, 28. Each of the retractors 22, 24 comprises a retractor blade 66 and a securing element 68 having a square internal profile 70.

The square internal profile 70 of the securing element 68 allows it to be slidably engaged on the square outer profile of the movable member 62 thereby preventing rotation of the retractor relative to the movable member 62.

The movable member 62 and the fixed member 52 are provided with respective interlocking formations 72 in the forms of serrations, to prevent relative rotation of the movable member 62 and the fixed member 52 when they are engaged with each other.

The blades 66 of the first and second retractors 22, 24 extend longitudinally from the securing element 68 and are curved around the longitudinal axis of the blade 66. This provides a convex surface for engagement against soft tissue in the body, thereby reducing damage to such tissue.

Referring back to Figs 1 and 2, when the bolt 54 tightens the movable member 62 against the fixed member 52, the interlocking formations 72 interlock with one another thereby preventing relative rotation.

Urging means in the form of a spring 74 urges the bolt 54 in the direction indicated by the arrow C in Fig 2, thereby urging the movable member 62 against the fixed member 52. Thus, by unscrewing the bolt 54, the movable member 62 can be moved away from the fixed member 52 thereby disengaging the interlocking formations 72 from each other and allowing pivotal movement of the movable member 62 relative to the fixed member 52. This allows the retractor 22 to be pivoted and, thus, positioned at the appropriate angle.

The second retractor 24 is mounted on a second mounting arrangement 28. The second mounting arrangement 28 comprises the securing assembly 38.

The second mounting arrangement 28 comprises a second securing assembly 39 which consists of the same features and operates in the same way as the first securing assembly 38 of the first mounting arrangement 26.

The second securing assembly 39 differs from the first securing assembly 38 in that the second securing assembly 39 is fixedly attached to the second side 16 by an elongate bar 80 extending therefrom. The bar 80 is fixedly attached to the second side 16 of the support member 12 by suitable bolts 82.

The third and fourth sides of 80, 20 of the support member 12 are provided with outwardly facing serrations 84 which allow a holding member 86 to be mounted in a desired position therealong. The holding member 86 comprises upstanding projections 88 to which a chain 90 can be secured.

The chain 90 is attached to the respective Hohman retractor 100, as shown in Fig 5.

Fig 5 shows two Hohman retractors, which could be used in surgery. Each of the Hohman retractors 100 comprises a main portion 102 and a soft tissue engaging portion 104 extending from the main portion 102. At the opposite end of the Hohman retractors 100 there is provided a securing portion 106 of a J shape having at its free end a securing part 108 to which the chain 90 can be attached to secure the Hohman retractor 102 to the holding member 86.

First removable handles 110 are provided on each of the first and second retractors 22, 24, to pivotally manipulate the retractors 22, 24 to the desired position.

Second removable handles 112 can be provided on the Hohman retractors 100 to position the Hohman retractors 100 in the desired position.

In use, the support member 12 is arranged over the hip of the patient undergoing the operation, and an incision is made through the space 36 defined by the support member 12.

The use of the present embodiment provides the advantage that the incision need only be two or three centimetres in length, rather than, as with prior art surgical procedures ten to fifteen centimetres in length.

Suitable first and second retractors 22, 24 are mounted on the first and second mounting arrangements 26, 28 to retract the soft tissue while the initial stages of the operation take place. In order to expose the acetabulum, the first and second retractors 22, 24, having longer blades, are provided to carry out the second stage of the surgical process.

The soft tissue surrounding the acetabulum can be retracted by the use of the Hoh man retractors 102.

As can be seen from Figs 3 and 4 the first and second retractor 22, 24 are provided with spikes or serrations, or other formations at the free end to engage the various parts of the body being operated upon and hold such parts in place during the operation.

There is thus described a simple and effective surgical apparatus which can be used in hip replacement or resurfacing surgery allowing minimally invasive hip surgery to be carried out thereby reducing the recovery time of the patient.

Various modifications can be made without departing from the scope of the invention. For example, the handles used on the first and second retractors 22, 24 and on the Hohman retractors 102 could be of a different shape.

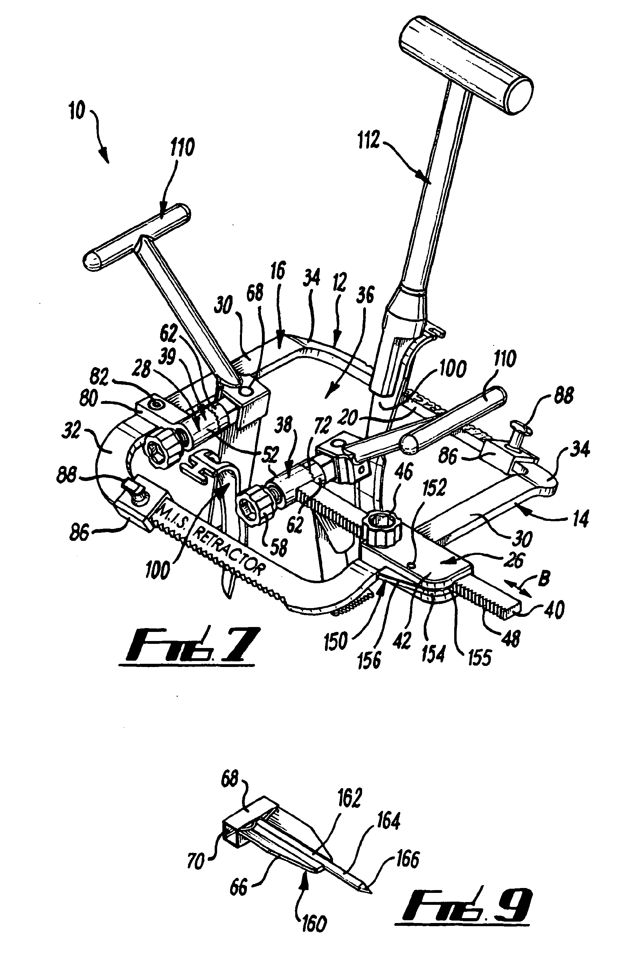

Referring to Fig 7, there is shown a second embodiment of a surgical apparatus 10, which comprises most of the features of the first embodiment shown in Fig 1 and described above. In Fig 7, these features have been designated with the same reference numerals as in Fig 1, and operate in the same way as the corresponding features shown in Fig 1.

The chains 90 securing the Hohman retractors 100 to the frame 12 are omitted from Fig 7 for clarity.

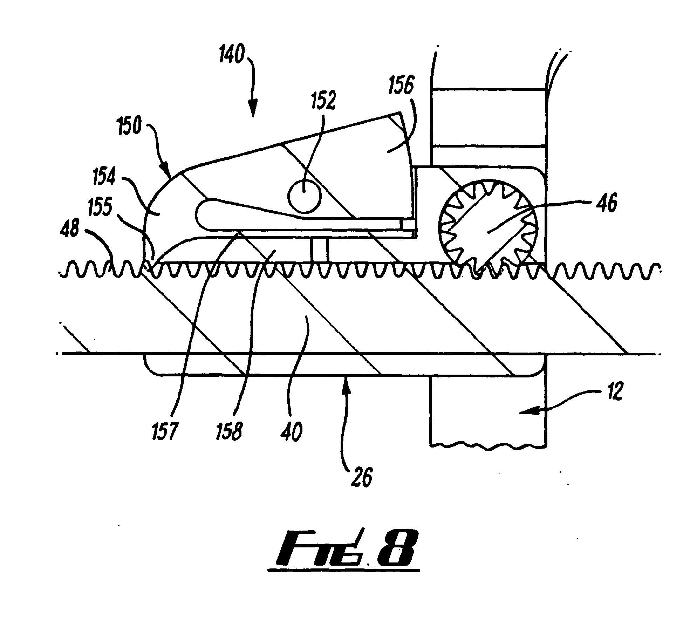

The second embodiment of the surgical apparatus 10 shown in Fig 7 differs from the first embodiment shown in Fig 1 in that the locking member is omitted. Instead, a rocker assembly 140 is provided, which comprises depressible member in the form of a rocker button 150. The rocker assembly 140 is shown in more detail in Fig 8

The rocker button 150 is pivotally mounted in the housing 42 by a pivot member 152. The rocker button 150 has a first region 154 on one side of the pivot, and a second region 156 on the opposite side of the pivot 152. The first region 154 includes an engaging element 155 to engage the teeth 48 of the rack 40, as shown in Fig 8. In Fig 8, the rocker button 150 is in its locking position, preventing any movement of the rack 40 by the pinion 46.

An urging arrangement in the form of a leaf spring 157 is provided integrally on the rocker button 150. The leaf spring 157 extends from the first region 154 of the rocker button 150 along a reaction portion 158 of the first mounting arrangement 26.

When the rocker button 150 is depressed by pressing on the second region 156, the engaging element 155 is pivoted away from the teeth 48 of the rack 40, thereby disengaging the engaging element from the teeth 48 and moving the rocker button 150 to a release position.

The leaf spring 157 pushes against the reaction portion 158 and urges the rocker button to the locking position. Thus, when the user lets go of the rocker button 150, the leaf spring 157 pushes against the reaction portion 158 to urge the rocker button 150 towards the locking position to engage the teeth 48.

Fig 9 shows a third retractor 160, which comprises the securing element 68 having the square internal profile 70. The third retractor 160 also includes a blade 66. A spike 162 extends from the securing element 68 along the blade 66 to project therefrom at a projecting region 164. The spike 162 terminates at its free end at a point 166.

Whilst endeavouring in the foregoing specification to draw attention to those features of the invention believed to be of particular importance it should be understood that the Applicant claims protection in respect of any patentable feature or combination of features hereinbefore referred to and/or shown in the drawings within the scope to the claims.

## Claims

1. A surgical apparatus (10) comprising a support member (12) having first and second opposite sides (14,16); first and second retractors (22, 24) on the support member; a first mounting arrangement (26) on the first side, and a second mounting arrangement (28) on the second side; the first mounting arrangement being configured to mount the first retractor on the first side, wherein at least the first retractor is movable towards and away from the second retractor, and the second mounting arrangement being configured to mount the second retractor on the second side; and each mounting arrangement comprising a securing assembly (38, 39) to secure the first and second retractors to the first and second mounting arrangements respectively; wherein each securing assembly comprises a fixed member (52); a movable member (62) to which the first or second retractor can be secured; a fastener (54) extending through the fixed member, the fastener cooperatively engaging the fixed member; **characterised by** urging means (74) on the fastener to urge the movable member into engagement with the fixed member.

2. A surgical apparatus according to Claim 1, wherein the fastener (54) comprises a head (58) and a shaft (56) having a threaded region, and the movable member (62) is threaded to threadably engage the shaft, and the movable member has a securing formation to secure the first or second retractor onto the movable member.

3. A surgical apparatus according to Claim 1 or 2, wherein the first and second retractors (22, 24) face each other, and the first and second retractors are pivotally movable relative to the support member (12).

4. A surgical apparatus according to Claim 1, 2 or 3, wherein the support member (12) defines an access region through which surgery can be effected, the support member (12) surrounding the afore said access region, and the retractors (22,24) extending through the access region.

5. A surgical apparatus according to according to any preceding Claim, wherein each of the first and second sides (14, 16) comprises a central region (30) and opposite end regions (32, 34) extending transverse to the central region, the opposite end regions of each of the first and second sides being angled relative to the central region at an angle in the range of 20° to substantially 30° to the central region.

6. A surgical apparatus according to Claim 5, wherein the support member (12) comprises opposite third and fourth sides (18, 20), the third and fourth sides extending between the first and second sides (14, 16) to connect the first side to the second side, thereby providing a generally rectangular profile of the support member, the third and fourth sides each including a movable holding member (86) to secure a respective surgical member (100) thereto, and the third and fourth sides further including serrations (84) to hold the holding member in a selected position on the respective third or fourth side.

7. A surgical apparatus according to Claim 6, wherein each holding member (86) includes a securing formation to which a connecting element can be attached to connect the respective surgical member (100) to the holding member, the securing formation comprising an upstanding projection (88), and the connecting element is elongate, and each surgical member (100) comprises a respective manipulating member (110) to move the respective surgical member to its desired position.

8. A surgical apparatus according to any preceding Claim, wherein the movable member (62) has interlocking formations, and the fixed member (52) has corresponding interlocking formations to co-operatively engage the interlocking formations on the movable member.

9. A surgical apparatus according to any preceding Claim, wherein the movable member (62) includes a securing formation to secure the respective first or second retractor (22, 24) onto the movable member, the first and second retractors each comprising a securing element (68) slidably mounted on the securing formation to engage the respective movable member, and wherein the securing formation and the securing element are correspondingly configured to substantially prevent relative rotation of the movable member and the respective retractor.

10. A surgical apparatus according to Claim 9, wherein the securing formation comprises a generally rectangular outer profile (64) of the movable member (62), and the securing element (68) has a generally rectangular inner profile (70), said inner profile of the securing element corresponding to the outer profile of the securing formation.

11. A surgical apparatus according to any preceding Claim, wherein each of the movable members (62) is pivotally movable relative to the respective first or second fixed members (52) to arrange the respective first or second retractors (22, 24) at a desired angle relative to the support member (12).

12. A surgical apparatus according to any preceding Claim, wherein the first mounting arrangement (26) comprises a displacement mechanism to move the first retractor (22) towards and away from the second retractor (24), the displacement mechanism comprising a displacement member to which the respective fixed member (52) is attached, the displacement member being elongate and comprising a rack (40), and the displacement mechanism further including a housing (42), through which the displacement member movably extends.

13. A surgical apparatus according to Claim 12, wherein the first mounting arrangement (26) includes a driver to drive the displacement member, the driver comprising a rotatable head and a cog in engagement with the displacement member.

14. A surgical apparatus according to Claim 12 or 13, wherein a locking member (50) is provided to lock the displacement member to the housing (42), the locking member comprising a threaded member threadably receivable in the housing to engage the mounting member, and the locking member further includes a rotatable head mounted on the threaded member to rotate the threaded member.

15. A surgical apparatus according to Claim 12 or 13, wherein a locking member is provided to lock the displacement member to the housing (42), the locking member comprising a depressible member (150) for engaging the displacement member, the depressible member being movable between a locking position and a release position by pressing thereon.

16. A surgical apparatus according to Claim 15, wherein the depressible member (150) can pivot about a pivot member (152) between the locking and the release positions, the depressible element being depressible at a first region (154) on one side of the pivot member to move the depressible element to the locking position, and the depressible element is depressible at a second region (156) on the opposite side of the pivot member to move the depressible member to the release position.

## Patentansprüche

1. Eine chirurgische Vorrichtung (10), die ein Stützelement umfasst (12), welches wiederum folgende Bestandteile umfasst: erste und zweite gegenüberliegende Seiten (14, 16); erste und zweite Retraktoren (22, 24) auf dem Stützelement; die erste Befestigungsvorrichtung (26) auf der ersten Seite sowie eine zweite Befestigungsvorrichtung (28) auf der zweiten Seite; die erste Befestigungsvorrichtung ist dahingehend konfiguriert, dass der erste Retraktor auf der ersten Seite aufgesetzt werden kann, wobei sich zumindest der erste Retraktor hin zum und weg vom zweiten Retraktor bewegen lässt, und die zweite Befestigungsvorrichtung ist dahingehend konfiguriert, dass der zweite Retraktor auf der zweiten Seite aufgesetzt werden kann; und jede Befestigungsvorrichtung besteht aus einer Sicherungsvorrichtung (38, 39), mit der der erste und der zweite Retraktor auf der ersten bzw. zweiten Befestigungsvorrichtung sicher befestigt werden; wobei jede Sicherungsvorrichtung folgendes umfasst: ein festes Element (52); ein bewegliches Element (62), an das der erste oder zweite Retraktor befestigt werden können; einen Halter (54), der durch das feste Element gezogen wird; der Halter ist dabei zusammenwirkend mit dem festen Element verbunden; was wiederum durch Belastungsmittel (74) auf dem Halter **gekennzeichnet** ist, welche das bewegliche Element mit dem festen Element zusammenwirken lassen.

2. Eine chirurgische Vorrichtung gemäß Patentanspruch 1, bei der der Halter (54) ein Kopfstück (58) und einen Schaft (56) umfasst, der über ein Gewinde verfügt; das bewegliche Element (62) wird eingezogen, so dass es durch das Gewinde mit dem Schaft zusammenwirkt; das bewegliche Element verfügt ferner über eine Sicherungsvorrichtung, mit der der erste oder der zweite Retraktor auf das bewegliche Element angebracht werden.

3. Eine chirurgische Vorrichtung gemäß Patentanspruch 1 oder 2, bei der sich der erste und der zweite Retraktor (22, 24) gegenüber stehen, und bei der der erste und der zweite Retraktor im Verhältnis zum Stützelement (12) zentral beweglich sind.

4. Eine chirurgische Vorrichtung gemäß Patentanspruch 1, 2 oder 3, bei der das Stützelement (12) einen Zugriffsbereich definiert, durch den der chirurgische Eingriff vorgenommen werden kann, das Stützelement (12), welches den vorstehend genannten Zugriffsbereich umgibt, sowie die Retraktoren (22, 24), die sich durch den Zugriffsbereich erstrecken.

5. Eine chirurgische Vorrichtung gemäß den vorstehenden Patentansprüchen, bei der jede der ersten und zweiten Seiten (14, 16) einen zentralen Bereich (30) sowie die gegenüber liegenden Bereiche (32, 34) umfasst, die sich quer durch den zentralen Bereich erstrecken; die gegenüberliegenden Endbereiche jeder der ersten und zweiten Seiten haben dabei im Verhältnis zum zentralen Bereich einen Winkel zwischen 20° und überwiegend 30° zum zentralen Bereich.

6. Eine chirurgische Vorrichtung gemäß Patentanspruch 5, bei der das Stützelement (12) gegenüberliegende dritte und vierte Seiten (18, 20) umfasst, wobei sich die dritten und vierten Seiten zwischen den ersten und zweiten Seiten (14, 16) erstrecken, um die erste Seite mit der zweiten Seite zu verbinden; **dadurch** entsteht ein im Allgemeinen rechteckiges Profil des Stützelements, wobei die dritten und vierten Seiten jeweils ein bewegliches Halteelement umfassen (86), mit dem das entsprechende chirurgische Element (100) darauf befestigt wird, und die dritten und die vierten Seiten umfassen außerdem Zahnreihen (84), mit denen das Halteelement in einer ausgewählten Position auf der entsprechenden dritten oder vierten Seite gehalten wird.

7. Eine chirurgische Vorrichtung gemäß Patentanspruch 6, bei der jedes Halteelement (86) eine Sicherungsvorrichtung umfasst, an die ein Verbindungselement angebracht werden kann, mit dem das entsprechende chirurgische Element (100) mit dem Halteelement verbunden werden kann, wobei die Sicherungsvorrichtung einen hochstehenden Vorsprung (88) umfasst; ferner ist das Verbindungselement verlängert und jedes chirurgische Element (100) hat ein entsprechendes Verschiebeelement (110), mit dem das entsprechende chirurgische Element in die gewünschte Position gebracht werden kann.

8. Eine chirurgische Vorrichtung gemäß den vorstehenden Patentansprüchen, bei denen das bewegliche Element (62) über Kopplungsausformungen verfügt und das feste Element (52) ebenfalls über entsprechende Kopplungsausformungen verfügt, die mit den Kopplungsausformungen des beweglichen Elements zusammenwirkend ineinander greifen.

9. Eine chirurgische Vorrichtung gemäß den vorstehenden Patentansprüchen, bei der das bewegliche Element (62) eine Sicherungsvorrichtung umfasst, mit der der erste bzw. der zweite Retraktor (22, 24) auf dem beweglichen Element befestigt werden, wobei der erste und zweite Retraktor beide ein Sicherungselement (68) umfassen, das verschoben werden kann und auf der Sicherungsvorrichtung angebracht ist, so dass es mit dem entsprechenden beweglichen Element zusammenwirken kann, und bei der die Sicherungsvorrichtung und das Sicherungselement dahingehend konfiguriert wurden, dass diese eine relative Rotation des beweglichen Elements und des entsprechenden Retraktors nachhaltig vermeiden.

10. Eine chirurgische Vorrichtung gemäß Patentanspruch 9, bei der die Sicherungsvorrichtung ein im Allgemeinen rechteckiges Außenprofil (64) des beweglichen Elements (62) umfasst; ferner hat das Sicherungselement (68) im Allgemeinen ein rechteckiges Innenprofil (70); dieses Innenprofil des Sicherungselements entspricht dem Außenprofil der Sicherungsvorrichtung.

11. Eine chirurgische Vorrichtung gemäß den vorstehenden Patentansprüchen, bei der die einzelnen beweglichen Elemente (62) im Verhältnis zu den ersten bzw. den zweiten festen Elementen (52) zentral beweglich sind, so dass die ersten bzw. zweiten Retraktoren (22, 24) im gewünschten Winkel zum Stützelement (12) angebracht werden können.

12. Eine chirurgische Vorrichtung gemäß den vorstehenden Patentansprüchen, bei der die erste Befestigungsvorrichtung (26) einen Verdrängungsmechanismus umfasst, mit dem der erste Retraktor (22) zum zweiten Retraktor (24) hin und davon weg bewegt werden kann, wobei der Verdrängungsmechanismus wiederum ein Verdrängungselement umfasst, an das das entsprechende feste Element (52) angebracht wird, wobei das Verdrängungselement verlängert ist und ein Rahmengestell umfasst (40); ferner beinhaltet der Verdrängungsmechanismus auch ein Gehäuse (42), durch das sich das Verdrängungselement beweglich erstreckt.

13. Eine chirurgische Vorrichtung gemäß Patentanspruch 12, bei der die erste Befestigungsvorrichtung (26) einen Antrieb für das Verdrängungselement umfasst, wobei der Antrieb einen Rotationskopf und ein Zahnrad umfasst, die mit dem Verdrängungselement zusammengreifen.

14. Eine chirurgische Vorrichtung gemäß Patentanspruch 12 oder 13, bei der ein Schließelement (50) enthalten ist, mit dem das Verdrängungselement an das Gehäuse festgeschlossen wird (42); das Schließelement umfasst ein Gewindeelement, das mittels Gewinde in das Gehäuse eingebracht werden kann, um das Befestigungselement zu aktivieren; das Schließelement umfasst ferner einen Rotationskopf, der auf dem Gewindeelement befestigt ist und mit dem das Gewindeelement gedreht wird.

15. Eine chirurgische Vorrichtung gemäß Patentanspruch 12 oder 13, bei der ein Schließelement enthalten ist, mit dem das Verdrängungselement an das Gehäuse (42) festgeschlossen wird; das Schließelement umfasst ein Drückelement (150), mit dem das Verdrängungselement betätigt werden kann, wobei das Drückelement zwischen einer Schließposition und einer Freigabeposition durch Drücken dieses Elements bewegt werden kann.

16. Eine chirurgische Vorrichtung gemäß Patentanspruch 15, bei der das Drückelement (150) sich zwischen den Schließ- und den Freigabepositionen um ein Drehelement (152) drehen kann, wobei das Drückelement in einem ersten Bereich (154) auf einer Seite des Drehelements betätigt werden kann, um das Drückelement in Schließposition zu bringen, das Drückelement kann ferner in einem zweiten Bereich (156) auf der gegenüberliegenden Seite des Drehelements betätigt werden, um das Drückelement in Freigabeposition zu bringen.

## Revendications

1. Appareil chirurgical (10) comprenant un élément de support (12) doté de premier et deuxième côtés opposés (14,16 ); de premier et deuxième rétracteurs (22, 24) sur l'élément de support ; d'un premier agencement de montage (26) sur le premier côté et un deuxième agencement de montage (28) sur le deuxième côté ; le premier agencement de montage étant configuré de manière à être monté sur le premier rétracteur sur le premier côté, dans lequel au moins le premier rétracteur est mobile en direction et à distance du deuxième rétracteur, et le deuxième agencement de montage étant configuré pour être monté sur le deuxième rétracteur sur le deuxième côté ; et chaque agencement de montage comprenant un ensemble de fixation (38, 39) pour attacher les premier et deuxième rétracteurs aux premier et deuxième agencements de montage respectivement ; dans lequel chaque ensemble de fixation comprend un élément fixe (52) ; un élément mobile (62) auquel le premier ou deuxième rétracteur peut être attaché ; une pièce de fixation (54) s'étendant à travers l'élément fixe, la pièce de fixation s'engageant en coopération avec l'élément fixe ; **caractérisé par** des moyens d'engagement (74) sur la pièce de fixation pour pousser l'élément mobile en coopération avec l'élément fixe.

2. Appareil chirurgical selon la Revendication 1, dans lequel la pièce de fixation (54) comprend une tête (58) et un arbre (56) doté d'une zone filetée, et l'élément mobile (62) est fileté de manière à se visser avec la partie filetée de l'arbre, et l'élément mobile est doté d'un ensemble de fixation pour attacher le premier ou le deuxième rétracteur à l'élément mobile.

3. Appareil chirurgical selon la Revendication 1 ou 2, dans lequel les premier et deuxième rétracteurs (22, 24) se trouvent face-à-face et les premier et deuxième rétracteurs sont mobiles de façon pivotante relativement à l'élément de support (12).

4. Appareil chirurgical selon la Revendication 1, 2 ou 3, dans lequel l'élément de support (12) définit une zone d'accès à travers laquelle l'acte chirurgical peut être réalisé, l'élément de support (12) entourant la zone d'accès prémentionnée et les rétracteurs (22,24) s'étendant à travers la zone d'accès.

5. Appareil chirurgical selon l'une quelconque des revendications précédentes, dans lequel chacun des premier et deuxièmes côtés (14, 16) comprend une zone centrale (30) et des zones d'extrémité opposées (32, 34) s'étendant de manière transversale à la zone centrale, les zones d'extrémité opposées de chacun des premier et deuxième côtés étant angulaires par rapport à la zone centrale à un angle se situant dans la plage de 20° à sensiblement 30° par rapport à la région centrale.

6. Appareil chirurgical selon la Revendication 5, dans lequel l'élément de support (12) comprend des troisième et quatrième côtés opposés (18, 20), ces derniers s'étendant entre les premier et deuxième côtés (14, 16) pour rattacher le premier côté au deuxième côté, procurant ainsi un profil généralement rectangulaire à l'élément de support, les troisième et quatrième côtés intégrant un élément de retenue (86) pour y fixer un élément chirurgical respectif (100) et les troisième et quatrième côtés comportant en outre des dentelures (84) pour maintenir l'élément de retenue en une position choisie sur le troisième ou quatrième côté respectif.

7. Appareil chirurgical selon la Revendication 6, dans lequel chaque élément de retenue (86) comporte un ensemble de fixation auquel peut s'attacher un élément de liaison pour rattacher les éléments chirurgicaux respectifs (100) à l'élément de retenue, l'ensemble de fixation comprenant une projection verticale (88), et l'élément de liaison est allongé, et chaque élément chirurgical (100) comporte un élément de manipulation respectif (110) pour déplacer l'élément chirurgical respectif à sa position souhaitée.

8. Appareil chirurgical selon l'une quelconque des revendications précédentes, dans lequel l'élément mobile (62) possède des ensembles d'emboîtage et l'élément fixe (52) possède des ensembles d'emboîtage correspondants afin d'engager en coopération les ensembles d'emboîtage sur le membre mobile.

9. Appareil chirurgical selon l'une quelconque des revendications précédentes, dans lequel l'élément mobile (62) comporte un ensemble de fixation pour attacher le premier ou deuxième rétracteur respectif (22, 24) sur l'élément mobile, les premier et deuxième rétracteurs comportant chacun un élément de fixation (68) monté de manière coulissante sur l'ensemble de fixation pour s'engager sur l'élément mobile respectif, et dans lequel l'ensemble de fixation et l'élément de fixation sont conçus de manière correspondante pour empêcher sensiblement la rotation relative de l'élément mobile et du rétracteur respectif.

10. Appareil chirurgical selon la Revendication 9, dans lequel l'ensemble de fixation présente un profil extérieur généralement rectangulaire (64) de l'élément mobile (62), et l'élément de fixation (68) possède un profil intérieur généralement rectangulaire (70), ledit profil intérieur de l'élément de fixation correspondant au profil extérieur de l'ensemble de fixation.

11. Appareil chirurgical selon l'une quelconque des revendications précédentes, dans lequel chacun des éléments mobiles (62) est mobile de manière pivotante relativement au premier ou deuxième élément fixe respectif (52) afin d'agencer le premier ou deuxième rétracteur respectif (22,24) à un angle souhaité relativement à l'élément de support (12).

12. Appareil chirurgical selon l'une quelconque des revendications précédentes, dans lequel le premier agencement de montage (26) comprend un mécanisme de déplacement de manière à déplacer le premier rétracteur (22) en direction et à distance du deuxième rétracteur (24), le mécanisme de déplacement comprenant un élément de déplacement auquel l'élément fixe respectif (52) est attaché, l'élément de déplacement étant allongé et doté d'une crémaillère (40), et le mécanisme de déplacement intégrant en outre un logement (42), à travers lequel l'élément de déplacement s'étend de manière mobile.

13. Appareil chirurgical selon la Revendication 12, dans lequel le premier agencement de montage (26) comporte un arbre d'entraînement pour entraîner l'élément de déplacement, l'arbre d'entraînement comportant une tête rotative et un pignon en engagement avec l'élément de déplacement.

14. Appareil chirurgical selon la Revendication 12 ou 13, dans lequel est prévu un élément de verrouillage (50) afin de verrouiller l'élément de déplacement au logement (42), l'élément de verrouillage comportant un élément fileté vissé dans le boîtier pour coopérer avec l'élément de montage, et l'élément de verrouillage comporte en outre une tête rotative montée sur l'élément fileté pour faire tourner l'élément fileté.

15. Appareil chirurgical selon la Revendication 12 ou 13, dans lequel est prévu un élément de verrouillage pour verrouiller l'élément de déplacement au logement (42), l'élément de verrouillage comportant un élément dépressible (150) pour coopérer avec l'élément de déplacement, l'élément dépressible étant mobile entre une position de verrouillage et une position de dégagement par une pression sur celui-ci.

16. Appareil chirurgical selon la Revendication 15, dans lequel l'élément dépressible (150) peut pivoter autour d'un organe de pivotement (152) situé entre les positions de verrouillage et de dégagement, l'élément dépressible étant dépressible au niveau d'une première zone (154) d'un côté de l'organe de pivotement afin de déplacer l'élément dépressible à la position de verrouillage et l'élément dépressible est dépressible au niveau d'une deuxième zone (156) du côté opposé de l'organe de pivotement de manière à déplacer l'élément dépressible à la position de dégagement.
